(19) Europäisches Patentamt
European Patent Office
Office européen des brevets

(11) EP 0 544 091 B2

(12) NEUE EUROPÄISCHE PATENTSCHRIFT

(45) Veröffentlichungstag und Bekanntmachung des Hinweises auf die Entscheidung über den Einspruch:
**28.08.2002 Patentblatt 2002/35**

(45) Hinweis auf die Patenterteilung:
**08.03.1995 Patentblatt 1995/10**

(21) Anmeldenummer: **92117580.8**

(22) Anmeldetag: **15.10.1992**

(51) Int Cl.[7]: **B01J 31/40**, B01J 31/24, C07C 45/50

(54) **Verfahren zur Reaktivierung wasserlöslicher Hydroformylierungskatalysatoren**

Reactivation process for water soluble hydroformylation catalysts

Procédé de réactivation de catalyseurs d'hydroformylation soluble dans l'eau

(84) Benannte Vertragsstaaten:
**AT BE CH DE DK ES FR GB IT LI LU NL SE**

(30) Priorität: **24.10.1991 DE 4135050**

(43) Veröffentlichungstag der Anmeldung:
**02.06.1993 Patentblatt 1993/22**

(73) Patentinhaber: **Celanese Chemicals Europe GmbH**
**60439 Frankfurt am Main (DE)**

(72) Erfinder:
- **Konkol, Werner, Dr. Dipl.-Chem.**
  **W-4200 Oberhausen (DE)**
- **Bahrmann, Helmut, Dr. Dipl.-Chem.**
  **W-4236 Hamminkeln (DE)**
- **Herrmann, Wolfgang A., Prof. Dr. Dipl.-Chem.**
  **W-8051 Giggenhausen (DE)**
- **Kohlpaintner, Christian, Dipl.-Chem.**
  **W-8209 Stephanskirchen (DE)**

(56) Entgegenhaltungen:
**EP-A- 0 019 296**
**EP-A- 0 357 997**
**FR-A- 2 489 308**
**GB-A- 2 092 907**
**US-A- 2 627 354**
**US-A- 4 283 304**
**US-A- 4 605 708**
**US-A- 4 935 550**

EP 0 544 091 B2

## Beschreibung

Die Erfindung betrifft ein Verfahren zur Wiederherstellung der Wirksamkeit teilweise deaktivierter, wasserlöslicher Hydroformylierungskatalystoren.

Es ist bekannt, durch Umsetzung von Olefinen mit Kohlenmonoxid und Wasserstoff (Hydroformylierung) Aldehyde und Alkohole herzustellen, die ein Kohlenstoffatom mehr als das Ausgangsolefin enthalten. Die Reaktion wird durch Hydridometallcarbonyle, vorzugsweise solcher der Metalle der VIII. Gruppe des Periodensystems, katalysiert. Neben Kobalt, das als Katalysatormetall in großem Umfang technische Anwendung findet, hat in letzter Zeit Rhodium zunehmend Bedeutung gewonnen. Im Gegensatz zu Kobalt gestattet Rhodium, die Reaktion bei niedrigem Druck durchzuführen; darüber hinaus werden aus geradkettigen, endständigen Olefinen vorzugsweise n-Aldehyde und nur in untergeordnetem Maße iso-Aldehyde gebildet. Schließlich ist auch die Hydrierung der Olefine zu gesättigten Kohlenwasserstoffen in Gegenwart von Rhodium-Katalysatoren deutlich niedriger als bei Anwendung von Kobalt-Katalysatoren.

Bei den in der Technik eingeführten Verfahren ist der Rhodium-Katalysator ein durch zusätzliche Liganden, insbesondere tertiäre organische Phosphine oder Phosphite modifiziertes Hydridorhodiumcarbonyl. Meist liegen die Liganden im Überschuß vor, so daß das Katalysatorsystem aus Komplexverbindung und freiem Ligand besteht. Der Einsatz der beschriebenen Rhodium-Katalysatoren ermöglicht es, die Hydroformylierungsreaktion bei Drücken unter 30 MPa durchzuführen.

Schwierigkeiten verursachen bei diesem Verfahren jedoch die Abtrennung der Reaktionsprodukte und die Wiedergewinnung der im Reaktionsprodukt homogen gelösten Katalysatoren. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d.h. Olefine mit bis zu etwa 8 Kohlenstoffatomen im Molekül, beschritten werden. Außerdem hat sich gezeigt, daß die thermische Belastung des Destillationsgutes auch zu erheblichen Katalysatorverlusten durch Zersetzung der Rhodium-Komplexverbindungen führt.

Die geschilderten Mängel werden durch Anwendung von Katalysatorsystemen vermieden, die in Wasser löslich sind. Derartige Katalysatoren sind z.B. in der DE-PS 26 27 354 beschrieben. Die Löslichkeit der Rhodium-Komplexverbindungen wird hierbei durch Verwendung sulfonierter Triarylphosphine als Komplexbestandteil erreicht. Die Abtrennung des Katalyators vom Reaktionsprodukt nach Beendigung der Umsetzung erfolgt bei dieser Verfahrensvariante einfach durch Dekantieren von wäßriger und organischer Phase, d.h. ohne Destillation und damit ohne zusätzliche thermische Verfahrensschritte. Ein weiteres Merkmal dieser Arbeitsweise ist, daß mit hoher Selektivität aus geradkettigen endständigen Olefinen n-Aldehyde und nur in ganz untergeordnetem Maße iso-Aldehyde gebildet werden. Als Komplexbestandteile wasserlöslicher Rhodium-Komplexverbindungen werden vorzugsweise sulfonierte, daneben auch, wie z.B. aus der DE-A1 31 35 127 bekannt ist, carboxylierte Triarylphosphine eingesetzt.

Bei kontinuierlicher Arbeitsweise oder bei wiederholtem Einsatz derselben Katalysatorlösung nimmt im Laufe der Zeit die Aktivität des Katalysatorsystems ab und ebenso seine Fähigkeit, mit hoher Selektivität unverzweigte Aldehyde zu bilden. Dieser Aktivitäts- und Selektivitätsverlust hat verschiedene Gründe. Zu den Ursachen gehören Katalysatorgifte wie Eisencarbonyl, das sich durch Einwirkung von Synthesegas auf die Tranportleitungen oder das Reaktormaterial bildet oder auch höhersiedende Kondensationsprodukte, die aus den Aldehyden entstehen. Selektivitätsmindernd wirkt auch die Abnahme des Verhältnisses von Phosphin zu Rhodium bei längerer Anwendung des Katalysatorsystems, eine Folge von Abbau- und Oxidationsprozessen, denen die sulfonierten oder carboxylierten Phosphine unterworfen sind. Im Verlauf dieser Reaktionen werden aus den sulfonierten Verbindungen z.B. Phosphinoxide, Phosphinsulfide, aromatische Sulfonsäuren, $\alpha$-Hydroxybutyldisulfophenyl-phosphinoxid, disulfophosphinige Säure und Disulfophenylphosphinsäure, jeweils in Form ihrer Salze, gebildet.

Weder Phosphinoxide und Phosphinsulfide, noch die Salze aromatischer Sulfonsäuren und der Disulfophenylphosphinsäure sind allein oder zusammen mit Rhodium katalytisch wirksam. Das gleiche gilt auch für Clusterverbindungen des Rhodiums, die sich nach langen Reaktionszeiten aus der katalytisch wirksamen Rhodium-Komplexverbindung bilden können. Um die ursprüngliche Aktivität und Selektivität des Katalysatorsystems wiederzuerhalten, kann man die unwirksam gewordenen Katalysatorbestandteile, ohne sie aus dem Reaktionsgemisch zu entfernen, bis zur Einstellung der Anfangskonzentration durch frische Rhodiumverbindung und/oder frisches Phosphin ersetzen.

Schließlich sind als Umwandlungsprodukte auch noch sulfonierte bzw. carboxylierte Alkyl-arylphosphine zu erwähnen, die hemmend auf die Hydroformylierungsreaktion einwirken. Diese gemischten, aliphatisch-aromatischen Phosphine bilden sich im Laufe der Umsetzung der Olefine mit Kohlenmonoxid und Wasserstoff aus dem sulfonierten bzw. carboxylierten Triarylphosphin durch Austausch von Arylresten gegen Alkylgruppen, wobei die Alkylgruppen sich von dem Olefin ableiten, das hydroformyliert wird. So entsteht z.B. bei der Reaktion von Propylen in Gegenwart sulfonierten oder carboxylierten Triphenylphosphins disulfoniertes bzw. dicarboxyliertes n-Propyl-diphenylphosphin Die gemischten, aliphatisch-aromatischen Phosphin ergeben mit Rhodium katalytisch inaktive Komplexverbindungen mit der Folge, daß Reaktionsgeschwindigkeit und Katalysatoraktivität deutlich abnehmen.

Es bestand daher die Aufgabe, die inaktivierend wirkenden Alkyl-arylphosphine möglichst selektiv aus dem Reaktionsgemisch zu entfernen.

Erfindungsgemäß wird diese Aufgabe gelöst durch ein Verfahren zum Regenerieren wasserlöslicher Katalysatorsysteme, die in einer Hydroformulierungsreaktion unter Verwendung wässriger Katalysatorlösungen gebraucht wurden und Rhodium und Verbindungen der allgemeinen Formel I

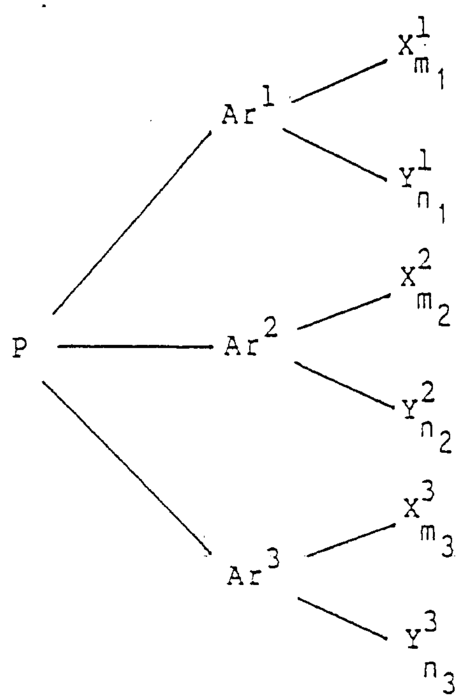

(I)

wobei $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine geradkettige oder verzweigte Alkoxygruppe mit 1 bis 4 C-Atomen, ein Halogenatom, die OH-, CN-, $NO_2$- oder $R^1R^2N$-Gruppe, in der $R^1$ und $R^2$ für jeweils eine geradkettige oder verzweigte Alkylgruppe mit 1 bis 4 C-Atomen stehen, bedeuten, $X^1$, $X^2$ und $X^3$ ein Carboxylat-($-COO^-$-) und/oder ein Sulfonat-($-SO_3^-$-)Rest ist, $m_1$, $m_2$, $m_3$ gleiche oder verschiedene ganze Zahlen von 0 bis 3 sind, wobei mindestens eine Zahl $m_1$, $m_2$, $m_3$ gleich oder größer als 1 ist und $n_1$, $n_2$, $n_3$ gleiche oder verschiedene ganze Zahlen von 0 bis 5 sind, komplexgebunden und gegebenenfalls im Überschuß enthalten. Dieses Verfahren ist dadurch gekennzeichnet, daß man den wäßrigen Lösungen der Katalysatorsysteme zur Umsetzung sulfonierter oder carboxylierter Alkyl-arylphosphine Maleinsäure, Fumarsäure oder olefinisch ungesättigte Verbindungen der allgemeinen Formel II

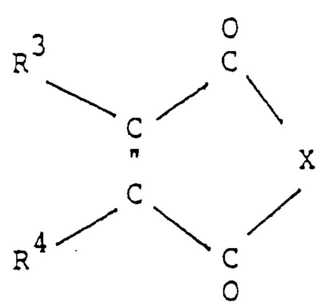

II

in der X für O, S, N-$R^5$ oder P-$R^5$ steht und $R^3$, $R^4$ und $R^5$ gleich oder verschieden sind und Wasserstoff, einen Alkyl- oder Arylrest bedeuten, zusetzt.

Überraschenderweise führt das neue Verfahren zu einer deutlichen Wiederbelebung selbst solcher Katalysatoren, die über längere Zeiträume verwendet wurden. Aktivität und Selektivität erlangen durch die erfindungsgemäße Maßnahme vielfach ihre ursprünglichen Werte wieder. Besonders hervorzuheben ist, daß die Reaktivierung des Katalysators während seines Einsatzes erfolgen kann, so daß eine Abtrennung der Katalystorlösung vom Reaktionsgemisch oder eine Unterbrechung der laufenden Synthese nicht erforderlich ist. Überdies können die Umwandlungsprodukte aus dem wasserlöslichen Alkyl-arylphosphin und der wasserlöslichen olefinischen Verbindung in der wäßrigen Lösung verbleiben. Dadurch unterscheidet sich die neue Arbeitsweise vorteilhaft von der Hydroformylierung in homogener Phase unter Verwendung von Rhodium/Arylphosphin-Katalysatoren. Auch bei dieser Variante der Oxosynthese können unerwünschte Umwandlungsprodukte der lipophilen Arylphosphine durch Umsetzung mit bestimmten olefinisch ungesättigten Verbindungen unschädlich gemacht werden (vgl. EP 00 19 296 A1). Es ist jedoch erforderlich, die Umwandlungsprodukte aus dem Reaktionsgemisch durch Extraktion mit Wasser zu entfernen. Dieses Verfahren setzt also die Abtrennung des Katalysatorsystems vom Reaktionsgemisch voraus, es kann daher nicht in einer laufenden Anlage durchgeführt werden.

Ein unerwarteter Nebeneffekt der erfindungsgemäßen Arbeitsweise ist die Erhöhung der Reaktionsgeschwindigkeit bei der Hydroformylierung höherer Olefine, d. h. Olefine mit 9 und mehr Kohlenstoffatomen, unter Verwendung von Katalysatorlösungen, die aus der Umsetzung wasserlöslicher Alkyl-arylphosphine mit wasserlöslichen olefinisch ungesättigten Verbindungen hervorgehende Verbindungen enthalten. Möglicherweise sind die neu entstandenen Verbindungen Phasentransferreagentien (Lösungsvermittler), die die Löslichkeit der höheren Olefine in der wäßrigen Phase verbessern und damit die Reaktion beschleunigen.

Zur Regenerierung von Katalysatorsystemen nach dem erfindungsgemäßen Verfahren werden üblicherweise die in der Synthese eingesetzten wäßrigen Katalysatorlösungen verwendet. Sie enthalten im allgemeinen Rhodium in Konzentrationen von 450 bis 800 Gew.-ppm und wasserlösliche Phosphine in Konzentrationen von 25 bis 30 Gew.-%, jeweils bezogen auf die wäßrige Lösung. Das Rhodium liegt als Carbonylwasserstoffverbindung, die in komplexer Bindung zusätzlich Phosphin enthält, vor. Nichtgebundene Phosphine sind in der Lösung als Salze gelöst, vorzugsweise als Alkali-, Ammonium- oder quaternäre Ammoniumsalze. Sie entsprechen der oben wiedergegebenen allgemeinen Formel I. Phosphine, in denen $Ar^1$, $Ar^2$ und $Ar^3$ gleich sind und Phenyl oder Naphthyl bedeuten, $X^1$, $X^2$, $X^3$ für einen Carboxylat-Rest ($-COO^-$) oder einen Sulfonat-Rest ($-SO_3^-$) stehen, $m_1$, $m_2$ und $m_3$ jeweils 0 oder 1 sind mit der Maßgabe, daß die Summe von $m_1$, $m_2$ und $m_3$ mindestens 1 ist und $n_1$, $n_2$ und $n_3$ gleich 0 sind, werden bevorzugt eingesetzt. Beispiele für solche Phosphine sind Tri(m-sulfophenyl)-phosphin, Phenyl-di(m-sulfophenyl)-phosphin und Diphenyl-(m-sulfophenyl)-phosphin. Daneben sind in der Lösung die weiter oben näher charakterisierten Umwandlungsprodukte der Phosphine, z.B. Phosphinoxide und Phosphinsulfide und gegebenenfalls noch andere Stoffe, wie Lösungsvermittler, enthalten. Die Bildung von Nebenprodukten erfolgt unabhängig von den individuellen olefinisch ungesättigten Verbindungen, die unter Einwirkung der wäßrigen Katalysatorlösung hydroformyliert wurden.

Bei den als störend erkannten Alkyl-arylphosphinen handelt es sich um Verbindungen, die einen oder zwei Alkylreste und 2 bzw. 1 sulfonierte oder carboxylierte Arylgruppe enthalten. Auch diese Phosphine sind in der Lage, mit Rhodium Komplexverbindungen zu bilden, doch hat sich gezeigt, daß sie katalytisch nicht oder nur wenig wirksam sind. Möglicherweise beruht die Inaktivität der Alkylarylphoshine auf ihrer im Vergleich zu Arylphosphinen höheren Basizität. Entsprechend der angenommenen Bildungsweise stimmen die Arylreste in den gemischt aliphatisch-aromatischen Phosphinen mit denen überein, die in den Triarylphosphinen des Katalysatorsystems enthalten sind und die Anzahl der Kohlenstoffatome im Alkylrest ist gleich der Anzahl der Kohlenstoffatome im eingesetzten Olefin. Daher entstehen z.B. bei der Hydroformylierung von Propylen in Gegenwart von Tri(m-sulfophenyl)phosphin Propyl-di(m-sulfophenyl)phosphin und Dipropyl-(m-sulfophenyl)phosphin und bei der Hydroformylierung von Buten Phosphine, die neben einer bzw. zwei m-Sulfophenylgruppen zwei oder eine Butylgruppe enthalten.

Um die in der Katalysatorlösung enthaltenen Alkyl-arylphosphine unschädlich zu machen, setzt man sie mit olefinischen Verbindungen der allgemeinen Formel II

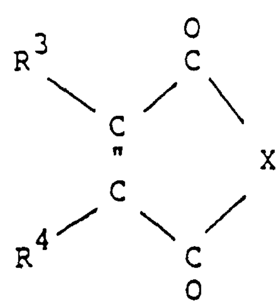

II

um. In Formel II steht X für O, S, $N-R^5$ oder $P-R^5$; $R^3$, $R^4$ und $R^5$ sind gleich oder verschieden und bedeuten einen Alkyl- oder Arylrest. Insbesondere sind $R^3$, $R^4$ und $R^5$ Wasserstoff, unverzweigte oder verzweigte $C_2$-$C_6$-Alkylreste

oder nichtsubstituierte oder substituierte Phenylreste.

Es ist nicht erforderlich, daß die olefinischen Verbindungen in Wasser leicht löslich sind. Es reicht aus, daß sie, wenn auch nur in geringer Menge, in die wäßrige Phase übergehen und in dem Maße, in dem sie durch die Reaktion verbraucht werden, wieder in diese Phase übertreten. Dennoch bevorzugt man in Wasser leicht und in organischen Medien schwer oder unlösliche Substanzen, um die Reaktion zu vereinfachen und zu beschleunigen und eine Anreicherung der olefinisch ungesättigten Verbindungen in der organischen Phase zu vermeiden. Als olefinisch ungesättigte Verbindungen bewährt haben sich Maleinsäure, Fumarsäure, Maleinsäureanhydrid, Maleinsäureimid, besonders geeignet sind Maleinsäure und Maleinsäureanhydrid. Die Verbindungen werden als solche verwendet oder, u. a. um ihre Dosierung zu vereinfachen, als Lösungen, zweckmäßig in Wasser oder in Lösungsmitteln, die mit Wasser mischbar sind. Die Konzentration der olefinisch ungesättigten Verbindungen in den Lösungen kann in weiten Bereichen variiert werden, bevorzugt werden Lösungen, die 0,1 bis 40 Gew.-%, insbesondere 0,1 bis 10 Gew.-% ungesättigte Verbindung (bezogen auf die Lösung) enthalten.

Die Umsetzung vollzieht sich in einfacher Weise durch Mischen von Katalysatorphase und olefinisch ungesättigter Verbindung. Temperatur und Druck sind für die Reaktion nicht kritisch, im allgemeinen arbeitet man bei Temperaturen von 0 bis 150°C, wobei Temperaturen von 100°C und mehr selbstverständlich die Anwendung erhöhten Druckes erfordern. Besonders einfach ist die Umsetzung unter den Bedingungen der Hydroformylierungsreaktion und parallel zu ihr im selben Reaktor vorzunehmen. Alkyl-arylphosphin und olefinische Verbindung reagieren miteinander im Molverhältnis 1 : 1. Dementsprechend muß man je mol des zu entfernenden Phosphins. seine Menge kann analytisch, z.B. durch HPLC-Analyse bestimmt werden, mindestens 1 mol olefinisch ungesättigte Verbindung anwenden. Zweckmäßig setzt man die olefinisch ungesättigte Verbindung im Überschuß ein, eine kritische obere Grenze gibt es nicht, doch empfiehlt es sich je mol Alkyl-arylphosphin, 15 bis 20 mol olefinisch ungesättigte Verbindung zu verwenden. Die Reaktivierung des Katalysatorsystems kann auch wiederholt erfolgen. Sowohl bei der Verwendung der olefinisch ungesättigten Verbindung im Überschuß als auch bei der wiederholten Reaktivierung der Katalysatorlösung ist zu beachten, daß die Umsetzung des wasserlöslichen Alkylarylphosphins nicht selektiv erfolgt, sondern daß stets auch in untergeordnetem Maße sulfoniertes oder carboxyliertes Triarylphosphin mitreagiert. Es kann daher erforderlich sein, von Zeit zu Zeit sulfoniertes oder carboxyliertes Triarylphosphin in der Katalysatorlösung zu ergänzen, um das optimale Verhältnis von Rhodium zu Phosph i n aufrechtzuerhalten.

In den folgenden Beispielen wird die Erfindung näher erläutert, sie ist aber selbstverständlich nicht auf die beschriebenen Ausführungsformen beschränkt.

Folgende Abkürzungen werden verwendet:

PDSPP : Di-Na-propyldisulfophenylphosphin
TPPDS : Di-Na-phenyldisulfophenylphosphin
TPPTS : Tri-Na-phenyltrisulfophenylphosphin
MSA : Maleinsäureanhydrid

Versuchsdurchführung

Die Versuche wurden in einer kontinuierlich arbeitenden, aus einem Rührreaktor, einem Phasentrenner und einer Produktvorlage mit Abgasregelung bestehenden Laborapparatur durchgeführt. Der Reaktor wurde mit 150 ml, der Phasentrenner mit 130 ml Katalysatorlösung befüllt. Die Katalysatorlösung enthielt etwa 300 Gew.-ppm Rh, das Molverhältnis Phosphor (III)/Rh variierte zwischen 100 : 1 (frische Katalysatorlösung) und 15 : 1 (gebrauchte Katalysatorlösung). Nach Einstellen der Reaktionsbedingungen, 122°C Reaktortemperatur, 5,0 MPa Synthesegasdruck (CO : $H_2$ = 1 : 1), wurden je nach Aktivität der eingesetzten Katalysatorlösung kontinuierlich 20 - 40 g Propylen in den Reaktor gepumpt.

Das in den Phasentrenner überlaufende Reaktionsgemisch (wäßrig-organisches Produkt, CO/$H_2$, Propylen) wurde getrennt und die sich absetzende wäßrige Katalysatorfö
sung kontinuierlich in den Reaktor zurückgeführt. In der Produktvorlage wurden Oxorohprodukt und Synthesegas voneinander geschieden und die hierbei anfallende wäßrige Lösung im geschlossenen Kreislauf in den Reaktor gepumpt. Der austretende Rohaldehyd wurde in der Produktvorlage gesammelt, seine Menge stündlich ermittelt.

Zur Beschreibung der Wirksamkeit der jeweiligen Katalysatorlösungen wurden die nachstehend definierten Größen "Aktivität" und "Produktivität" ermittelt.

$$\text{Aktivität: } \frac{\text{mol (n+i) Aldehyd}}{\text{g-Atom Rhodium . min}}$$

$$\text{Produktivität: } \frac{\text{g (n+i) Aldehyd}}{\text{cm}^3 \text{ Katalysatorlösung . h}}$$

Hierzu wurde stündlich die in der Produktvorlage angefallene Rohaldehydmenge und nach Beendigung des Versuchs die im Reaktor befindlichen Katalysatormenge gemessen.

Beispiele 1 - 3 (Tabelle 1)

Die Beispiele 1 - 3 zeigen die desaktivierende Wirkung von PDSPP auf eine frische, in der Hydroformylierung eingesetzte Rh/TPPTS-Katalysatorlösung. Bei Beispiel 1 handelt es sich um einen Bezugsversuch mit frischer Katalysatorlösung. Ihre (absolute) Aktivität liegt bei 15,8 und wird als relative Aktivität gleich 100 gesetzt. In den Beispielen 2 und 3 wurden der Katalyatorlösung zunehmende Mengen PDSPP zugesetzt. Als Orientierungsgröße dient das Molverhältnis (TPPTS + TPPDS)/PDSPP.

Tabelle 1

| Beispiel | 1 | 2 | 3 |
|---|---|---|---|
| | Na-TPPTS | Zugabe von 1,73 Mol % PDSPP | Zugabe von 7,4 Mol % PDSPP |
| A-Wert | 15,78 | 14,5 | 7,33 |
| P-Wert | 0,213 | 0,202 | 0,101 |
| P(III)/Rh-Verhältnis | 100 | 100 | 100 |
| (TPPTS+TPPDS):PDSPP | 252:0 | 240,7:1,4 = 172:1 | 225,4:10,2 = 22:1 |
| relative Aktivität | 100 | 92 | 46 |

Bei Zusatz von 1,7 mol % (bezogen auf P(III)-Gehalt) PDSPP zeigt sich im Rahmen der Schwankungsbreite noch kein Einfluß. Bei Erhöhung auf 7,4 mol %, dies entspricht einem Molverhältnis (TPPTS + TPPDS)/PDSPP von 22 : 1, ist jedoch ein eindeutiger stark negativer Einfluß zu erkennen.

Beispiele 4 und 5 (Tabelle 2)

Beispiel 4 als Bezugsversuch zeigt die A- und P-Werte einer gealterten Katalysatorlösung. Die relative Aktivität ist auf 41 % gefallen. Das Molverhältnis (TPPTS + TPPDS)/PDSPP beträgt 13 : 1.

Beispiel 5 verdeutlicht die Wirkung der Zugabe von Maleinsäureanhydrid als olefinisch ungesättigte Verbindung entsprechend der Erfindung. Die relative Aktivität steigt wieder auf 77,5 % an.

Tabelle 2

| Beispiel | 4 | 5 |
|---|---|---|
| | Altkontakt | Altkontakt + 0,4 Gew.-% MSA |
| A-Wert | 6,51 | 11,3 |
| P-Wert | 0,092 | 0,165 |
| P/Rh-Verhältnis | 32 | 17 |
| (TPPTS + TPPDS)/PDSPP | 94:7,4 = 13:1 | 59,5:1,6 = 37:1 |
| relative Aktivität | 41,2 | 77,5 |

Beispiele 6 - 11 (Tabelle 3)

Zur Ermittlung optimaler Werte wurde in den Beispielen 7 bis 11 die Maleinsäurezugabe zwischen 0,05 und 0,5 Gew.-%, bezogen auf die Altkatalysatorlösung, variiert.

Hierzu wurden 350 g Altkatalysatorlösung in einem 1 l Rührkolben unter Stickstoffatmosphäre jeweils mit der in Tabelle 3 angegebenen Menge MSA versetzt, 2 h unter Rückfluß erhitzt und anschließend durch HPLC-Analyse untersucht.

Tabelle 3

| Optimierung der Zugabe von MSA zur Altkatalysatorlösung | | | | | |
|---|---|---|---|---|---|
| Beispiel | 7 | 8 | 9 | 10 | 11 |
| MSA-Zugabe Gew.-% | - | 0,05 | 0,10 | 0,4 | 0,5 |
| mmol (TPPDS+TPPDS) | 88,5 | 87,0 | 76,7 | 59,9 | 47,7 |
| mmol PDSPP | 5,2 | 4,2 | 1,8 | 1,6 | 2,4 |
| $\frac{\text{TPPTS + TPPDS}}{\text{PDSPP}}$ | 17,02 | 20,71 | 42,6 | 37,4 | 19,9 |

Beispiel 7 gibt die Anteile TPPTS und TPPDS sowie PDSPP (jeweils in mmol) in der Katalysatorlösung wieder. Das Molverhältnis (TPPTS + TPPDS)/PDSPP dient wiederum als Optimierungsgröße. Je höher der Wert des Quotienten ist, desto größer ist der selektive Abbau des unerwünschten PDSPP. Wie man sieht, liegt das Optimum bei 0,1 Gew.-% MSA-Zugabe. Die folgenden Beispiele wurden deshalb bei dieser Zugabemenge durchgeführt.

<u>Beispiele 12 - 13 (Tabelle 4)</u>

| Beispiel | 4 | 12 | 13 |
|---|---|---|---|
| | Altkatalysator | Altkatalysator +0,1 Gew.% MSA | Altkatalysator +0,1 Gew.% MSA +7 Gew.% TPPTS |
| A-Wert | 6,5 | 16,9 | 21,9 |
| P-Wert | 0,092 | 0,256 | 0,335 |
| P/Rh-Verh. | 32 | 17 | ~ 25 |
| (TPPTS+ TPPDS)/ PDSPP | 13 : 1 | 28 : 1 | n.b. |
| rel. Aktivität (%) | 41,2 | 107 | 139 |

Wie Beispiel 12 zeigt, konnte die Aktivität im Rahmen der Schwankungsbreite wieder auf das Ausgangsniveau einer frischen Katalysatorlösung angehoben werden, obgleich die Gesamtsalzkonzentration (hierunter·wird die Gesamtheit der in der Lösung enthaltenen Salze verstanden) der Katalysatorlösung inzwischen von 15 auf etwa 30 % angestiegen ist.

Beispiel 13 macht deutlich, daß durch Erhöhung des P(III)-Gehaltes durch Frisch-TPPTS die Aktivität weit über das Ausgangsniveau angehoben werden kann. Die relative Aktivität steigt auf 139 %. Zur Erklärung dieser überraschenden Erscheinung wird angenommen, daß die in der Altkatalysatorlösung enthaltenen Abbauprodukte sowie die durch Maleinsäure gebildeten Phosphoniumsalze lösungsvermittelnde Eigenschaften besitzen, die ihre Wirkung aber erst dann entfalten können, wenn die aktivitätsmindernden Rh/PDSPP-Komplexe weitgehend abgebaut sind.

**Patentansprüche**

1. Verfahren zum Regenerieren wasserlöslicher Katalysatorsysteme, die in einer Hydroformylierungsreaktion unter Verwendung wässriger Katalysatorlösungen gebraucht wurden und Rhodium und Verbindungen der allgemeinen Formel I

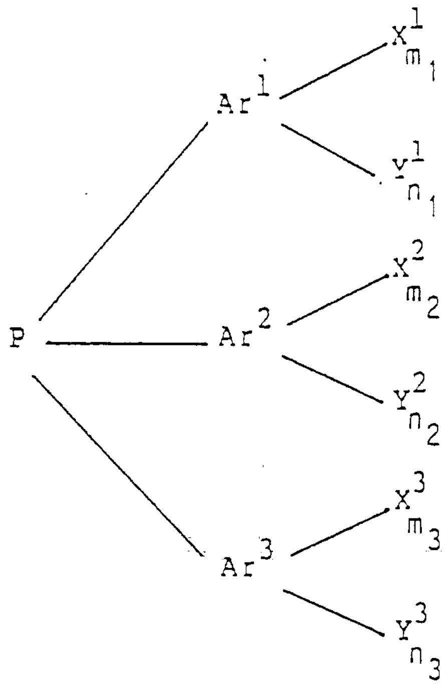

I

wobei $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine geradkettige oder verzweigte Alkoxygruppe mit 1 bis 4 C-Atomen, ein Halogenatom, die OH-, CN-, $NO_2$- oder $R^1R^2N$-Gruppe, in der $R^1$ und $R^2$ für jeweils eine geradkettige oder verzweigte Alkylgruppe mit 1 bis 4 C-Atomen stehen, bedeuten, $X^1$, $X^2$ und $X^3$ ein Carboxylat-($COO^-$-)und/oder ein Sulfonat-($SO_3^-$-)Rest ist, $m_1$, $m_2$, $m_3$ gleiche oder verschiedene ganze Zahlen von 0 bis 3 sind, wobei mindestens eine Zahl $m_1$, $m_2$, $m_3$ gleich oder größer als 1 ist und $n_1$, $n_2$, $n_3$ gleiche oder verschiedene ganze Zahlen von 0 bis 5 sind, komplexgebunden und gegebenenfalls im Überschuß enthalten, **dadurch gekennzeichnet, daß** man den wäßrigen Lösungen der Katalysatorsysteme zur Umsetzung sulfonierter oder carboxylierter Alkyl-arylphosphine Maleinsäure, Fumarsäure oder olefinisch ungesättigte Verbindungen der allgemeinen Formel II

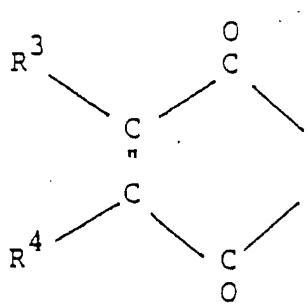

II

in der X für O, S, N-$R^5$ oder P-$R^5$ steht und $R^3$, $R^4$ und $R^5$ gleich oder verschieden sind und Wasserstoff, einen Alklylrest oder Arylrest bedeuten, zusetzt.

2. Verfahren nach Anspruch 1, **dadurch gekennzeichnet, daß** man je mol im Katalysatorsystem enthaltenen sulfonierten oder carboxylierten Alkyl-arylphosphins mindestens ein mol olefinisch ungesättigte Verbindung zusetzt.

3. Verfahren nach Anspruch 1 oder 2, **dadurch gekennzeichnet, daß** man je mol im Katalysatorsystem enthaltenen sulfonierten oder carboxylierten Alkyl-arylphosphins 15 bis 20 mol olefinisch ungesättigter Verbindung zusetzt.

4. Verfahren nach einem oder mehreren der Ansprüche 1 bis 3, **dadurch gekennzeichnet, daß** die olefinisch ungesättigte Verbindung Maleinsäureanhydrid oder Maleinsäure ist.

**5.** Verfahren nach einem oder mehreren der Ansprüche 1 bis 3, **dadurch gekennzeichnet, daß** die olefinisch ungesättigte Verbindung Maleinimid ist.

**6.** Verfahren nach einem oder mehreren der Ansprüche 1 bis 3, **dadurch gekennzeichnet, daß** die olefinisch ungesättigte Verbindung Fumarsäure ist.

**7.** Verfahren nach einem oder mehreren der Ansprüche 1 bis 6, **dadurch gekennzeichnet, daß** die olefinisch ungesättigte Verbindung dem Katalysatorsystem als wäßrige Lösung zugesetzt wird.

**8.** Verfahren nach Anspruch 7, **dadurch gekennzeichnet, daß** die wäßrige Lösung 0,1 bis 40 Gew.-%, insbesondere 0,1 bis 10 Gew.-% der olefinisch ungesättigten Verbindung, jeweils bezogen auf die Lösung, gelöst enthält.

**Claims**

**1.** A process for regenerating a water-soluble catalyst sytem used in a hydroformylation reaction using aqueous catalyst solutions which comprises rhodium and compounds of the formula I

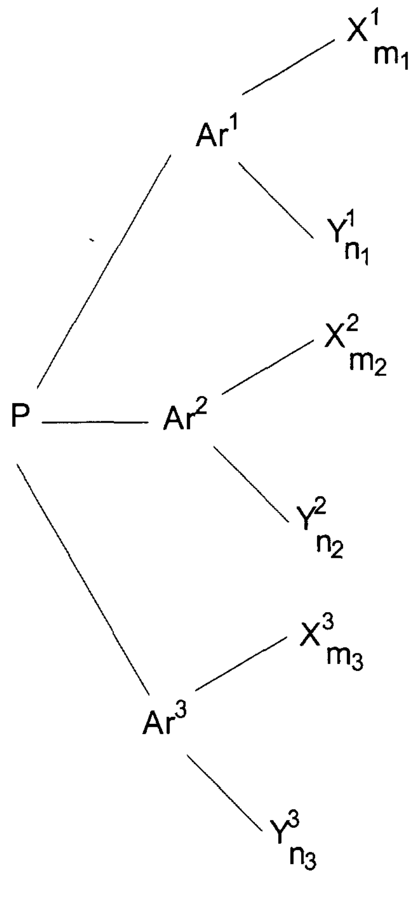

I

in which $Ar^1$, $Ar^2$ and $Ar^3$ are each a phenyl or naphthyl group, $Y^1$, $Y^2$ and $Y^3$ are each a straight-chain or branched alkyl group having 1 to 4 carbon atoms, a straight-chain or branched alkoxy group having 1 to 4 carbon atoms, a halogen atom or the OH, CN, $NO_2$ or $R^1R^2N$ group, in which $R^1$ and $R^2$ are each a straight-chain or branched alkyl group having 1 to 4 carbon atoms, $X^1$, $X^2$ and $X^3$ are a carboxylate ($-COO^-$) and/or a sulfonate ($-SO_3^-$) radical, $m_1$, $m_2$ and $m_3$ are identical or different integers from 0 to 3, at least one number $m_1$, $m_2$ or $m_3$ being equal to or greater than 1, and $n_1$, $n_2$ and $n_3$ are identical or different integers from 0 to 5, bonded in complex form and if appropriate in excess, which comprises adding maleic acid, fumaric acid or olefinically unsaturated compounds of the general formula II

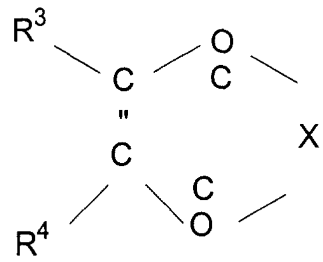

II

in which X is O, S, N-$R^5$ or P-$R^5$ and $R^3$, $R^4$ and $R^5$ are identical or different and are hydrogen, an alkyl radical or an aryl radical, to the aqueous solution of the catalyst system in order to react sulfonated or carboxylated alkyl-arylphosphines.

2. The process as claimed in claim 1, wherein at least one mol of olefinically unsaturated compound is added per mole of sulfonated or carboxylated alkyl-arylphosphine contained in the catalyst system.

3. The process as claimed in claim 1 or 2, wherein 15 to 20 mol of olefinically unsaturated compound are added per mole of sulfonated or carboxylated alkyl-arylphosphine contained in the catalyst system.

4. The process as claimed in one or more of claims 1 to 3, wherein the olefinically unsaturated compound is maleic anhydride or maleic acid.

5. The process as claimed in one or more of claims 1 to 3, wherein the olefinically unsaturated compound is maleimide.

6. The process as claimed in one or more of claims 1 to 3, wherein the olefinically unsaturated compound is fumaric acid.

7. The process as claimed in one or more of claims 1 to 6, wherein the olefinically unsaturated compound is added to the catalyst system as a aqueous solution.

8. The process as claimed in claim 7, wherein the aqueous solution contains 0.1 to 40% by weight, in particular 0.1 to 10% by weight, of the olefinically unsaturated compound, in each case b ased on the solution, in dissolved form.

**Revendications**

1. Procédé pour régénérer les systèmes de catalyseurs hydrosolubles utilisés dans une réaction d'hydroformylation avec utilisation de solutions aqueuses de catalyseur et qui contiennent du rhodium et des composés de formule générale I

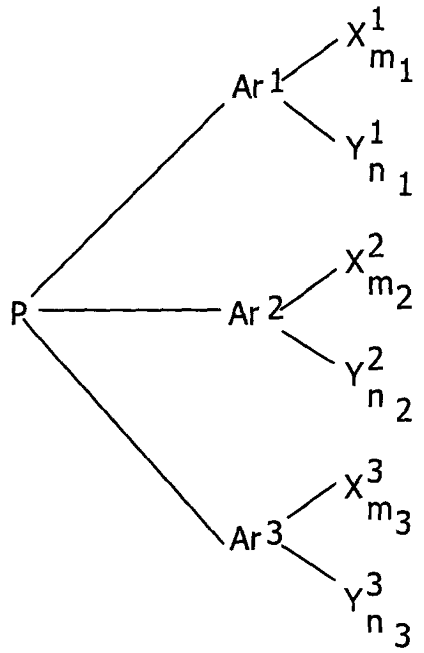

(I)

dans laquelle $Ar^1$, $Ar^2$, $Ar^3$ représentent à chaque fois un groupe phényle ou naphtyle, $Y^1$, $Y^2$, $Y^3$ représentent chacun un groupe alkyle linéaire ou ramifié ayant 1 à 4 atomes de carbone, un groupe alcoxy linéaire ou ramifié ayant 1 à 4 atomes de carbone, un atome d'halogène, le groupe -OH, -CN, $-NO_2$ ou $R^1R^2N$, dans lequel $R^1$ et $R^2$ représentent chacun un groupe alkyle, linéaire ou ramifié, ayant 1 à 4 atomes de carbone ; $X^1$, $X^2$ et $X^3$ représentent un reste carboxylate ($-COO^-$) et/ou un reste sulfonate ($-SO_3^-$) ; $m_1$, $m_2$, $m_3$ sont des nombres entiers égaux ou différents, valant de 0 à 3, au moins un nombre $m_1$, $m_2$ et $m_3$ étant égal ou supérieur à 1 ; et $n_1$, $n_2$ et $n_3$ sont des nombres entiers égaux ou différents valant de 0 à 5, en liaison complexe et éventuellement en excès, procédé **caractérisé en ce que**, pour convertir des alkyl-arylphosphines sulfonées ou carboxylées, on ajoute, aux solutions aqueuses des systèmes du catalyseur, de l'acide maléique, de l'acide fumarique ou des composés à insaturation éthylénique répondant à la formule générale II

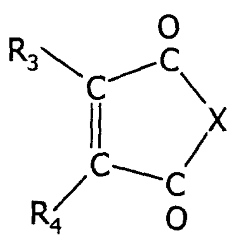

II

dans laquelle X représente O, S, $N-R^5$ ou $P-R^5$, et $R^3$, $R^4$ et $R^5$ sont identiques ou différents et représentent chacun un atome d'hydrogène, un reste alkyle ou un reste aryle.

**2.** Procédé selon la revendication 1, **caractérisé en ce que**, pour chaque mole d'alkyl-aryl-phosphine sulfonée ou carboxylée contenue dans le système du catalyseur, on ajoute au moins une mole de composé à insaturation oléfinique.

**3.** Procédé selon la revendication 1 ou 2, **caractérisé en ce que**, pour chaque mole d'alkyl-aryl-phosphine sulfonée ou carboxylée contenue dans le système du catalyseur, on ajoute 15 à 20 mol du composé à insaturation oléfinique.

**4.** Procédé selon une ou plusieurs des revendications 1 à 3, **caractérisé en ce que** le composé à insaturation oléfinique est l'anhydride de l'acide maléique ou l'acide maléique.

**5.** Procédé selon une ou plusieurs des revendications 1 à 3, **caractérisé en ce que** le composé à insaturation oléfinique est le maléinimide.

6. Procédé selon une ou plusieurs des revendications 1 à 3, **caractérisé en ce que** le composé à insaturation oléfinique est l'acide fumarique.

7. Procédé selon une ou plusieurs des revendications 1 à 6, **caractérisé en ce qu'**on ajoute au système du catalyseur le composé à insaturation oléfinique sous la forme d'une solution aqueuse.

8. Procédé selon la revendication 7, **caractérisé en ce que** la solution aqueuse contient en dissolution 0,1 à 40 % en poids, notamment 0,1 à 10 % en poids du composé à insaturation oléfinique (pourcentage) chaque fois rapporté à la solution.